(19) Europäisches Patentamt
European Patent Office
Office européen des brevets

(11) **EP 1 541 172 A1**

(12) **EUROPEAN PATENT APPLICATION**
published in accordance with Art. 158(3) EPC

(43) Date of publication:
**15.06.2005 Bulletin 2005/24**

(21) Application number: **03784490.9**

(22) Date of filing: **30.07.2003**

(51) Int Cl.[7]: **A61K 45/00**, A61K 31/454, A61P 1/00, A61P 29/00, A61P 43/00, A61P 1/04

(86) International application number:
**PCT/JP2003/009693**

(87) International publication number:
**WO 2004/014428 (19.02.2004 Gazette 2004/08)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **09.08.2002 JP 2002232561**

(71) Applicant: **Ajinomoto Co., Inc.**
**Tokyo 104-0031 (JP)**

(72) Inventors:
- **TOKUMASU, Munetaka c/o AJINOMOTO CO., INC. Kanagawa 210-0801 (JP)**
- **HASHIMOTO, Masaki c/o AJINOMOTO CO., INC. Kanagawa 210-0801 (JP)**
- **YANO, Tetsuo c/o AJINOMOTO CO., INC. Kanagawa 210-0801 (JP)**
- **MATSUMOTO, Hideki c/o AJINOMOTO CO., INC. Kanagawa 210-0801 (JP)**
- **FUJITA, Shinichi c/o AJINOMOTO CO., INC. Kanagawa 210-0801 (JP)**
- **SEKI, Tetsuya c/o AJINOMOTO CO., INC. Kanagawa 210-0801 (JP)**
- **ASARI, Sayaka c/o AJINOMOTO CO., INC. Kanagawa 210-0801 (JP)**
- **FUKUCHI, Naoyuki c/o AJINOMOTO CO., INC. Kanagawa 210-0801 (JP)**
- **TAKAHASHI, Kazuyoshi c/o AJINOMOTO CO., INC. Kanagawa 210-0801 (JP)**
- **SHOJI, Masataka c/o AJINOMOTO CO., INC. Kanagawa 210-0801 (JP)**

(74) Representative: **Nicholls, Kathryn Margaret et al**
**Mewburn Ellis LLP**
**York House**
**23 Kingsway**
**London WC2B 6HP (GB)**

(54) **REMEDY FOR INTESTINAL DISEASES AND VISCERAL PAIN**

(57) The present invention relates to a therapeutic agent for irritable bowel syndrome of diarrhea type, ulcerative colitis, visceral pain or abdominal pain, which contains a compound of the following formula and which has 5-HT7 receptor antagonistic effect or an analogue thereof; and this therapeutic agent has an excellent therapeutic effect and a high safety:

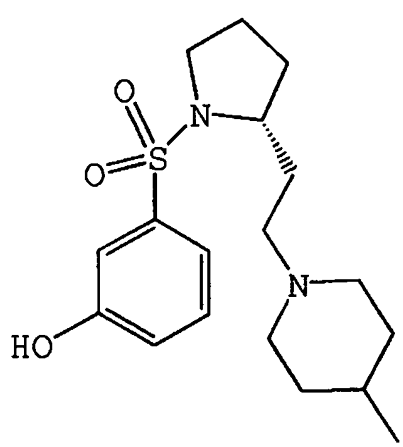

EP 1 541 172 A1

Printed by Jouve, 75001 PARIS (FR)

## Description

Background of the Invention:

The present invention relates to a therapeutic agent for intestinal diseases and visceral pain.

As the social environment is recently becoming complicated, a lot of people are exposed to excessive stress. Accordingly, patients suffering from irritable bowel syndrome with irregular bowel movement, abdominal pain, etc. as main symptoms are increasing in number. The irritable bowel syndrome is divided into the groups of diarrhea type, constipation type and alternative diarrhea/constipation type depending on the type of the irregular bowel movement. Medicines used for the treatment of the irritable bowel syndrome of diarrhea type are cholinergic blocking agents, 1 axatives, antidiarrheal agents, medicines for intestinal disorders, mucous membrane paralyzing agents, motor function regulators for the digestive tract, autonomic nerve regulators, herb medicines, anxiolytic agents, anti depressants, hypnotic, antipsychotic agents, etc.

On the other hand, ulcerative colitis can be mentioned as an intestinal disease which is increasing in number of the patients. Although the causes for this disease are supposed to be infection with bacteria and viruses, genetic causes, disorder of the blood vessels in the digestive tracts and lymph vessels, the exact causes have not yet been elucidated. For the treatment of ulcerative colitis, the following types of medicine are generally used: 5-aminosalicylic acid (trade name: Pentasa) and salazosulfapyridine (trade name: Salazopyrin) known to inhibit the production of inflammation-causing substances from leukocytes (such as inflammatory cytokine, leukotriene and active oxygen). For patients with moderate symptoms, synthetic adrenal cortex hormones such as predonisolone and betamethasone (trade names: Predonine and Rinderon), and cyclosporine (trade name: Sandimmun) as an immunosuppressive agent are generally used, and FK506 (trade name: Prograf) is now undergoing clinical testing.

Usually the visceral pain and abdominal pain are important biological signs for informing the pathological conditions of the visceral organs and abdominal region. The visceral pain and abdominal pain are not limited to the symptoms of the above-described intestinal diseases, i. e. irritable bowel syndrome of diarrhea type or ulcerative colitis, but there are sharp pains caused by sudden contraction or convulsion of tubular organs such as the stomach and gallbladder and inflammation of the peritoneum or pleura. For those symptoms, an antispasmodic agent or anti-inflammatory analgesic agent is used.

However, these medicines have only insufficient clinical effects and they are not always satisfactory from the viewpoint of side effects. Under these circumstances, the development of a medicine of a new type having an excellent therapeutic effect and free from side effects is demanded.

Serotonin (5-hydroxytryptamine, 5-HT) plays an important part in the physiological or behavioristic process. In particular, 90 % of serotonin in the living body is contained in intestinal chromaffin cells and, therefore, serotonin in the intestinal tract is physiologically and pathophysiologically very important. Up to now, 14 kinds of 5-HT receptors have been identified. In those receptors, 5-HT7 receptor is the most recently identified 5-HT receptor and in the peripheral tissues, the expression of 5-HT7 receptor in the coronary blood vessel and intestinal tract was reported [J. Biol. Chem., 268, 23422 (1993)].

5-HT7 receptor is conjugated with G protein (Gs) which accelerates the production of cyclic adenosine monophosphate (cAMP). Accordingly, cAMP concentration in the cells is increased through 5-HT7 receptor by the serotonin stimulation [J. Pharmacol. Exp. Ther., 287, 508 (1998)]. As the reports on the pharmacological effects related to this receptor, there can be mentioned reports relating to the relaxation reaction for intestinal smooth muscle [British J. Pharmacol., 128, 849 (1999)], possibility of the relation to the nociceptive sharp pain conduction [Life Sci., 21, 2279 (2002)] in the peripheral site and relation to the body temperature regulation and REM sleep [British J. Pharmacol., 139, 705 (2003)] in the central site. With the above-described background, it was disclosed that 5-HT7 receptor antagonist might be effective in the treatment of various diseases considered to be caused by an abnormalities of 5-HT central and peripheral 5-HT regulating function, such as mental disorders (e. g. manic-depressive psychosis, anxiety, schizophrenia, epilepsy, somnipathy , biological rhythm disorder and migraine), diseases in the cardiovascular system (e. g. hypertension) and systemic functional disorder of the digestive system [Official Gazette of Japanese Patent Kokai No. Hei 11-189585]. In this connection, the therapeutic effect of 5-HT7 receptor antagonist in rat models of middle cerebral artery occulution was also disclosed [WO 00/37082]. 5-HT7 receptor antagonist is expected to be effective in treating irritable bowel syndrome of diarrhea type, ulcerative colitis, visceral pain and abdominal pain which are diseases with digestive tract dyskinesia caused by the serotonin stimulation.

However, it has never been reported that 5-HT7 receptor antagonist is effective against these diseases. For example, compounds having antagonistic effect to 5-HT7 are disclosed in EP 0738513, Japanese Patent Kokai No. Hei 11-189585, WO 97/29097, WO 97/48681, WO97/49695, WO 98/00400, WO 99/24022, WO 99/31062, WO 99/33804, WO 00/00472, WO 00/56712, WO 00/59909, WO 00/69437, WO 00/73299, WO 01/29029, WO 01/57039, WO 01/85701, WO 02/18367, WO 02/36554, WO 02/36560, Trends Pharmacol. Sci. 21, 70 (2000), J. Med. Chem., 43, 342 (2000) and Bioorg. Med. Chem. Lett., 12, 3341 (2002). However, these publications do not disclose the advantageous effects of these compounds on the irritable bowel syndrome of diarrhea type, ulcerative colitis, visceral pain or abdominal pain. No example was reported on the therapeutic effects of these compounds on these diseases.

It is described in WO 02/62788 that compounds antagonistic to 5-HT7 will be usable for the treatment of various diseases such as pains including neuropathic pain, diabetic neuropathy and chronic backache, inflammations and irritable bowel syndrome in addition to the treatment of central nervous system diseases. However, it is not concretely disclosed therein that this medicine is effective against irritable bowel syndrome of diarrhea type, ulcerative colitis, visceral pain and abdominal pain.

WO 01/89546 discloses the effects of herb extracts on irritable bowel syndrome of diarrhea type. However, it does not clearly describe the therapeutic effects of compounds antagonistic to 5-HT7 for the following reasons: The extract contains not only a single active ingredient; the receptor antagonistic effect is as week as only about 50 % even at a high concentration (200 μg/ml); 15 kinds of the receptors have the receptor antagonistic effect of at least 50 %; and the receptor selectivity is unclear.

Disclosure of the Invention:

The object of the present invention is to provide a medicine used for the treatment of irritable bowel syndrome of diarrhea type, ulcerative colitis, visceral pain and abdominal pain and having a high safety.

After intensive investigations made for the purpose of developing a 5-HT7 receptor antagonist usable for the treatment of irritable bowel syndrome of diarrhea type, ulcerative colitis, visceral pain or abdominal pain, the inventors have found that the 5-HT7 receptor antagonist is usable as an effective therapeutic agent. The present invention has been completed on the basis of this finding.

Namely, the present invention provides a therapeutic agent for irritable bowel syndrome of diarrhea type, ulcerative colitis, visceral pain or abdominal pain, which contains 5-HT7 receptor antagonist or a pharmaceutically acceptable salt thereof as the active ingredient.

The present invention also provides the use of 5-HT7 receptor antagonist or a pharmaceutically acceptable salt thereof for the preparation of a therapeutic agent for irritable bowel syndrome of diarrhea type, ulcerative colitis, visceral pain or abdominal pain.

Brief Description of the Drawings:

Fig. 1 shows the length of the intestinal tract of each mouse of dextran sulfate sodium (DSS) model after the administration of test compound 1, predonisolone (PDL) and salazosulfapyridine (SASP). The number in the parentheses represents the number of mice.

Fig. 2 shows the infiltrated amout of Evans Blue of each mouse of dextran sulfate sodium (DSS) model after the administration of test compound 1, predonisolone (PDL) and salazosulfapyridine (SASP). The number in the parentheses represents the number of mice.

Fig. 3 shows the number of writhing of each mouse in acetic acid induced writhing model after the administration of test compound 1. The number in the parentheses represents the number of mice.

Best Mode for Carrying out the Invention:

The term "5-HT7 receptor antagonist" herein indicates a compound antagonising to 5-HT7 receptor. For exhibiting the therapeutic effect of 5-HT7 receptor antagonist in the present invention, it is desirable that this antagonist is a compound having a selective antagonistic effect on 5-HT7 receptor. Concretely, the expression "selectivity toward 5-HT7 receptor" herein indicates a receptor selectivity toward not only the other serotonin receptor subtypes but also deeply related physiologically active amine receptors, i. e. adrenaline receptor, muscarine receptor and dopamine receptor. The selectivity of 5-HT7 receptor toward the above-described other receptors is preferably at least 30 times higher, more preferably at least 100 times higher. For evaluating the receptor selectivity, an example of well-known methods is a receptor binding experiment wherein a ligand labeled with a radioisotope is used.

5-HT7 receptor antagonists may be compounds antagonistic to 5-HT7 receptors. For example, compounds described in, for example, EP 0738513, Japanese Patent Kokai No. Hei 11-189585, WO 97/29097, WO 97/48681, WO 97/49695, WO 98/00400, WO 99/24022, WO 99/31062, WO 99/33804, WO 00/00472, WO 00/56712, WO 00/59909, WO 00/69437, WO 00/73299, WO 01/29029, WO 01/57039, WO 01/85701, WO 02/18367, WO 02/36554, WO 02/36560 or WO 02/62788 are preferred as the therapeutic agents for the irritable bowel syndrome of diarrhea type, ulcerative colitis, visceral pain or abdominal pain.

Concretely, compounds represented by the following general formulae (I) to (VII) are preferred.

(I) Compounds represented by the following general formula (I);

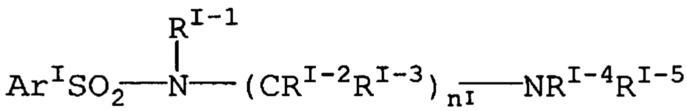 (I)

wherein:

$Ar^I$ represents a substituted or unsubstituted mono- or bicycloaromatic ring or heteroaromatic ring,
$R^{I-1}$ represents a lower alkyl,
$R^{I-2}$ and $R^{I-3}$ independently represent hydrogen or a lower alkyl, and
$R^{I-4}$ and $R^{I-5}$ independently represent hydrogen, a lower alkyl, an aryl-lower alkyl or an aryl or, $R^{I-4}$ and $R^{I-5}$ together form a substituted or unsubstituted, 5- to 8-membered heterocyclic ring with the nitrogen atom bonded thereto, which hetero ring may further contain a hetero atom selected from the group consisting of nitrogen, sulfur and oxygen, and $n^1$ represents 2 to 4.

(II) Compounds represented by the following general formula (II);

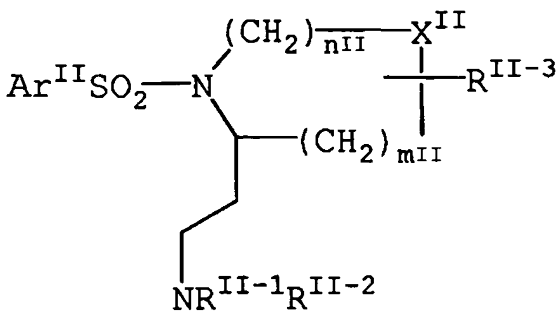 (II)

wherein:

$Ar^{II}$ represents a substituted or unsubstituted mono- or bicycloaromatic ring or heteroaromatic ring,
$R^{II-1}$ and $R^{II-2}$ independently represent hydrogen, a lower alkyl or an aryl-lower alkyl or, alternatively, $R^{II-1}$ and $R^{II-2}$ together form a substituted or unsubstituted, 5- to 7-membered heterocyclic ring with the nitrogen atom bonded thereto, which hetero ring may further contain a hetero atom selected from the group consisting of nitrogen, sulfur and oxygen, and the nitrogen atom may be substituted with hydrogen, a lower alkyl or $C_{3-7}$ cycloalkyl or with an aryl, a heteroaryl or an aryl-lower alkyl group,
$R^{II-3}$ represents hydrogen or a lower alkyl,
$X^{II}$ represents oxygen, sulfur or a bond,
$n^{II}$ represents 2 or 3, and
$m^{II}$ represents 1 or 2.

The optional substituent of the heterocyclic ring formed by $R^{II-1}$ and $R^{II-2}$ together is a lower alkyl, preferably one or two methyl and ethyl groups.
(III) Compounds represented by the following general formula (III);

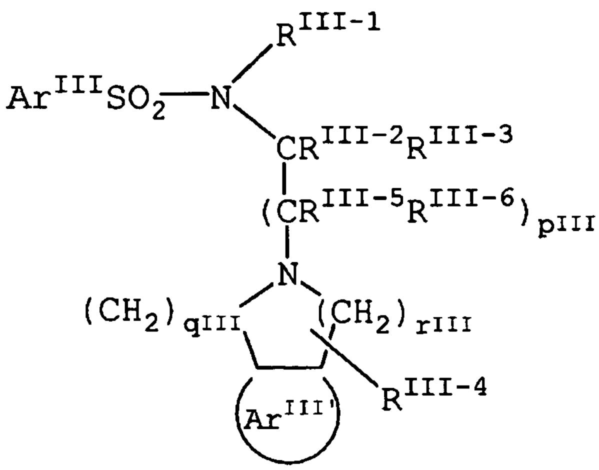

(III)

wherein:

$Ar^{III}$ represents a substituted or unsubstituted mono- or bicycloaromatic ring or heteroaromatic ring,
$Ar^{III'}$ represents a substituted or unsubstituted mono- or bicycloaromatic ring or a heteroaromatic ring,
$R^{III-1}$ represents a lower alkyl or $R^{III-1}$ and $R^{III-3}$ together form a 5- to 8-membered ring having 1 or 2 hetero atoms, which ring may be substituted with a lower alkyl,
$R^{III-2}$ represents hydrogen or a lower alkyl,
$R^{III-3}$ represents hydrogen or a lower alkyl, or $R^{III-3}$ and $R^{III-1}$ together form a 5 to 8-membered ring having 1 or 2 hetero atoms, which may be substituted with a lower alkyl,
$R^{III-4}$ represents hydrogen or a lower alkyl,
$R^{III-5}$ and $R^{III-6}$ independently represent hydrogen or a lower alkyl,
$p^{III}$ represents 1, 2 or 3,
$q^{III}$ represents 1 to 3, and
$r^{III}$ represents 1 or 2.

(IV) Compounds represented by the following general formula (IV);

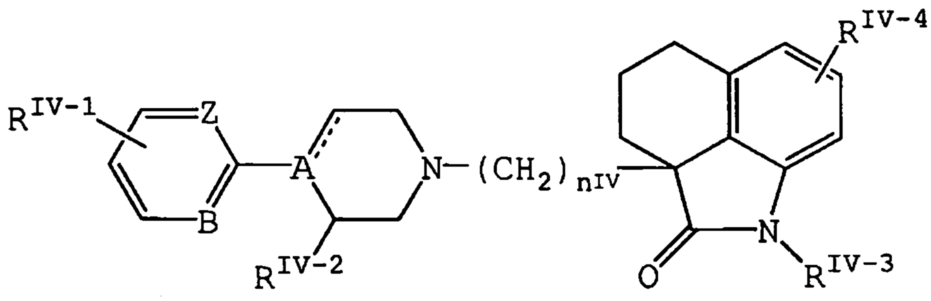

(IV)

wherein:

$Ar^{IV}$ represents N, CH, C having a double bond or $CR^{IV-5}$,
$B^{IV}$ and $Z^{IV}$ independently represent N or $CR^{IV-1}$, and when $B^{IV}$ and/or $Z^{IV}$ is N, $Ar^{IV}$ is N,
$R^{IV-1}$ represents hydrogen atom, a halogen atom, a lower alky, cyano, a trihalomethyl, hydroxyl, an alkoxyl, an alkylthio, an alkylsulfonyl, an alkylsulfonyl, an alkylcarbonyl, sulfamoyl, amino, a substituted amino, carbamoyl, an alkylcarbamoyl, an acyl or carboxyl,
$R^{IV-2}$ represents hydrogen or a lower alkyl,
$R^{IV-3}$ represents hydrogen, a lower alkyl or an aralkyl,

$R^{IV-4}$ represents hydrogen atom, a halogen atom, a lower alky, hydroxyl, an alkoxyl, an acyl, an alkoxycarbonyl, nitro, amino, a substituted amino, carbamoyl, an alkylcarbamoyl or an acyloxyl,
$R^{IV-5}$ represents a lower alkyl, cyano, carbamoyl, carboxyl, an acyl, an acyloxyl, an alkoxyl, an alkoxycarbonyl, a trihalomethyl or hydroxyl, and
$n^{IV-5}$ represents an integer of 2 to 6,

(V) Compounds represented by the following general formula (V);

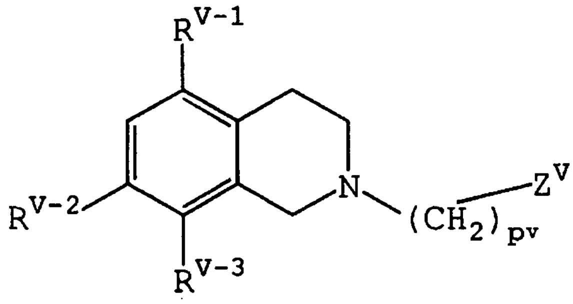

(V)

wherein:

$R^{V-1}$, $R^{V-2}$ and $R^{V-3}$ each represent hydrogen, a halogen, a lower alkyl or a lower alkoxyl, or $R^{V-2}$ and $R^{V-3}$ together form methylenedioxyl group,
$Z^V$ is any of the following groups a to d:

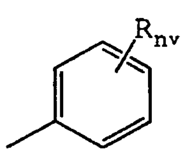

a

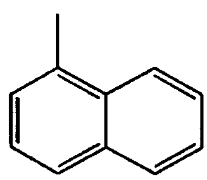

b

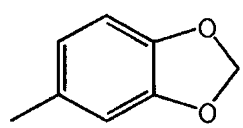

c

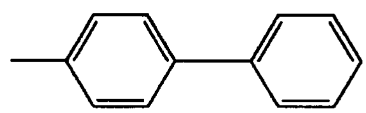

d

$R^V$ represents $CF_3$, a halogen, a lower alkoxyl, a lower alkyl or a lower alkyl-halogen,
$p^V$ represents 1 to 3, and
$n^V$ represents 0 to 5.

(VI) Compounds represented by the following general formula (VI);

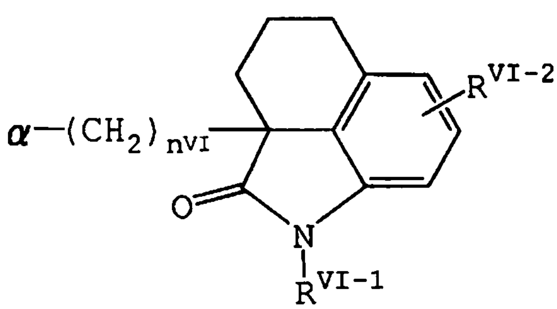

(VI)

wherein:

$R^{VI-1}$ represents hydrogen atom, a lower alkyl or an aralkyl,
$R^{VI-2}$ represents hydrogen atom, a halogen atom, a lower alkyl, hydroxyl, an alkoxyl, an acyl, an acyloxyl, an alkylcarbonyl, nitro, amino, a substituted amino, carbamoyl or an alkylcarbamoyl,
$n^{VI}$ represents an integer of 2 to 6,
$\alpha$ represents a group of the following formula (a), (b), (c), (d) or (e):

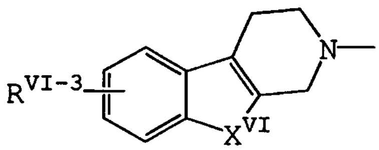
(a)

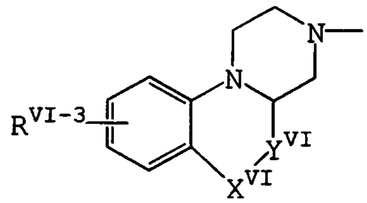
(b)

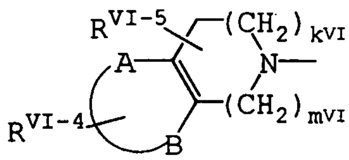
(c)

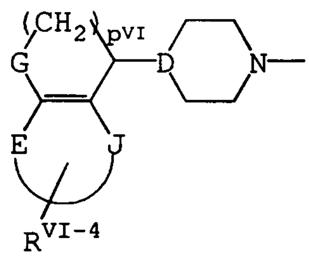
(d)

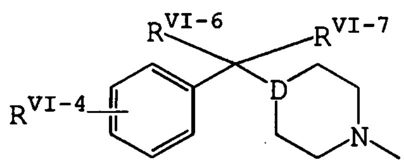
(e)

wherein, in formulae (a) and (b):

$R^{VI-3}$ represents hydrogen atom, a halogen atom, a lower alkyl, hydroxyl or an alkoxyl,
X represents $NR^{VI-10}$, $NCONR^{VI-11}R^{VI-12}$, S, SO, $SO_2$ or O,
$R^{VI-10}$ represents hydrogen atom, a lower alkyl, an alkenyl, an oxoalkyl, an aralkyl, a cyanoalkyl, a hydroxyalkyl, an alkoxyalkyl, an aminoalkyl, a substituted aminoalkyl, an alkoxycarbonylalkyl, a carbamoylalkyl, an alkylcarbamoylalkyl, an acyl or an alkoxycarbonyl,
$R^{VI-11}$ and $R^{VI-12}$ independently represent hydrogen atom or a lower alkyl,
$Y^{VI}$ represents methylene or carbonyl;

in formula c:

$R^{VI-4}$ represents hydrogen atom, a halogen atom, a lower alkyl, hydroxyl, cyano, a trihalomethyl, an alkoxyl, an alkylthio, an alkylsulfinyl, an alkylsulfonyl, an alkoxycarbonyl, sulfamoyl, amino, a substituted amino, carbamoyl, an alkylcarbamoyl, an acyl or carboxyl,
$R^{VI-5}$ represents hydrogen atom, a lower alkyl, hydroxyl, an alkoxyl, an acyl, phenyl or a substituted phenyl,
$k^{VI}$ represents 0 or an integer of 1 to 3,
$m^{VI}$ represents 0 or an integer of 1 to 3,
$A^{VI}$ and $B^{VI}$ each represent a group capable of forming benzene ring, thiophene ring, furan ring, imidazole ring or pyrazole ring through a double bond, with the proviso that $k^{VI}$ + $m^{VI}$ represent an integer of 1 to 3; in formulae (d) and (e):

$R^{VI-4}$ is as defined above,
$G^{VI}$ represents $CH_2$, S, O or C=O,
$D^{VI}$ represents CH or N,
$p^{VI}$ represents an integer of 1 to 3,

$E^{VI}$ and $J^{VI}$ independently represent a group capable of forming benzene ring or pyridine ring through a double bond,

$R^{VI-6}$ and $R^{VI-7}$ independently represent hydrogen atom, a lower alkyl, hydroxyl, an alkoxyl, an acyl, phenyl or a substituted phenyl.

(VII) Compounds represented by the following general formula (VII):

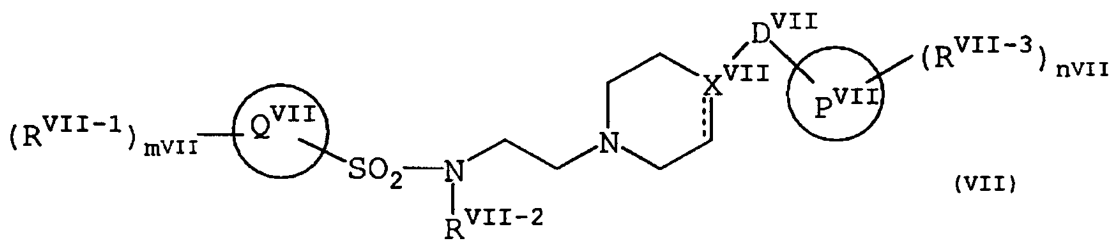

(VII)

wherein:

$Q^{VII}$ represents phenyl or thienyl,
$R^{VII-1}$ represents a halogen, hydroxyl, a $C_{1-6}$ alkyl, $CF_3$, $OCF_3$ or a $C_{1-6}$ alkoxyl,
$m^{VII}$ represents 0, 1, 2 or 3,
$R^{VII-2}$ represents a $C_{1-4}$ alkyl,
$X^{VII}$ represents nitrogen, carbon or CH,

- - - - represents a single bond when $X^{VII}$ is nitrogen or CH or it represents a double bond when $X^{VII}$ is carbon,

$D^{VII}$ represents a single bond, C=O, O or $CH_2$ with the proviso that when X is nitrogen atom, D is not oxygen atom,

$P^{VII}$ represents phenyl; naphthyl; a 5- or 6-membered heteroaryl ring having 1 to 3 hetero atoms selected from the group consisting of oxygen, nitrogen and sulfur; or a benzene-fused heteroaryl ring having 1 to 3 hetero atoms selected from the group consisting of oxygen, nitrogen and sulfur,

$R^{VII-3}$ represents a lower alkyl which may be substituted with $NR^{VII-4}R^{VII-5}$, an aryl, an aryl-lower alkyl, a lower alkoxyl, a lower alkylthio, cyano, hydroxyl, nitro, a halogen, $CF_3$, $C_2F_5$, $NR^{VII-4}R^{VII-5}$, $CONR^{VII-4}R^{VII-5}$, $NR^{VII-4}COR^{VII-5}$, $S(O)p^{VII}NR^{VII-4}R^{VII-5}$, CHO, $OCF_3$, $SCF_3$, $CH_2OR^{VII-6}$, $CO_2R^{VII-6}$ or $COR^{VII-6}$ (wherein $p^{VII}$ represents 0, 1 or 2, $R^{VII-4}$, $R^{VII-5}$ and $R^{VII-6}$ independently represent hydrogen, a lower alkyl, an aryl or an aryl-lower alkyl, and

$n^{VII}$ represents 0, 1, 2 or 3.

The compounds of the above formulae (I) to (VII) were disclosed in WO 97/29097, WO 97/48681, WO 97/49695, WO 98/00400, WO 99/24022, WO 99/33804 and WO 00/56712, respectively.

The following compounds (VIII) to (XXI) are also preferred; compounds of claim 1 of EP 0738513 (hereinafter referred to as "compounds (VIII)"), compounds of claim 1 of Japanese Patent Kokai No. Hei 11-189585 (compounds (IX)), compounds of claim 1 of WO 99/31062 (compounds (X)), compounds of claim 1 of WO 00/00472 (compounds (XI)), compounds of claim 1 of WO 00/59909 (compounds (XII)), compounds of claim 1 of WO 00/69437 (compounds (XIII)), compounds of claim 1 of WO 00/73299 (compounds (XIV)), compounds of claim 1 of WO 01/29029 (compounds (XV)), compounds of claim 1 of WO 01/57039 (compounds (XVI)), compounds of claim 1 of WO 01/85701 (compounds (XVII)), compounds of claim 1 of WO 02/18367 (compounds (XVIII)), compounds of claim 1 of WO 02/36554 (compounds (XIX)), compounds of claim 1 of WO 02/36560 (compounds (XX)) and compounds of claim 1 of WO 02/62788 (compounds (XXI)).

In these compounds, the compounds represented by the above formula (II) disclosed in WO 97/48681 are preferred as the therapeutic agents for the irritable bowel syndrome of diarrhea type, ulcerative colitis, visceral pain or abdominal pain. Particularly preferred 5-HT7 receptor antagonists for the irritable bowel syndrome of diarrhea type, ulcerative colitis, visceral pain or abdominal pain are (R)-3-(2-(2-(4-methylpiperidin-1-yl)-ethyl)-pyrrolidine-1-sulfonyl) phenol,(R)-1-bromo-3-(2-(2-(4-methylpiperidine-1-yl)-ethyl)pyrrolidine-1-sulfonyl)benzeneand(R)-2-(2-(4-methylpiperidin-1-yl)-ethyl)-1-(naphthalene-1-sulfonyl)pyrrolidine. These compounds are disclosed in Example 65, Example 51 and Example 45 in the above-described WO 97/48681. In addition, these compounds are also described as compounds

15, 12 and (R)-10, respectively in J. Med. Chem. 43, 342 (2000).

The term "lower" herein indicates those having 1 to 6 carbon atoms, preferably 1 to 4 carbon atoms.

The substituted or unsubstituted mono- or bicycloaromatic rings or heteroaromatic rings indicate monocyclic or bicyclic aromatic rings (the rings may contain nitrogen atom, oxygen atom or sulfur atom). The substituents of the mono- or bicycloaromatic rings or heteroaromatic rings are lower alkyl which may be substituted with $NR^7R^8$, a lower alkenyl, a lower alkynyl, a lower alkylthio, cyano, nitro, a halogen atom, $CF_3$, $C_2F_5$, $NR^7R^8$, $CONR^7R^8$, $NR^7COR^8$, S(O)p$N^7R^8$, CHO, $OCF_3$, $SCF_3$, $COR^9$, $CH_2OR^9$, $CO_2R^9$ or $OR^9$ (wherein p represents 1 or 2, $R^7$, $R^8$ and $R^9$ independently represent hydrogen, a lower alkyl, a substituted or unsubstituted aryl or a substituted or unsubstituted aryl-lower alkyl). There can be more than one substituent. The plural substituents may be the same or different from one another.

The substituted or unsubstituted mono- or bicycloaromatic rings or heteroaromatic rings are, for example, phenyl, naphthyl, pyridyl, pyrimidyl, pyridazinyl, pyrazinyl, imidazolyl, isoxazolyl, pyrazolyl, oxazolyl, thiazolyl, furyl, thienyl, pyrrolyl, triazolyl, tetrazolyl, isothiazolyl, quinolyl, isoquinolyl, quinazolinyl, phthalazinyl, indolyl, benzothiazolyl, benzoimidazolyl, benzofuranyl, indazolyl, isoindolyl and benzothienyl.

The aryl groups include phenyl and naphthyl.

The heteroaryl groups include, for example, pyridyl, pyrimidyl, pyridazinyl, pyrazinyl, imidazolyl, isoxazolyl, pyrazolyl, oxazolyl, thiazolyl, furyl, thienyl, pyrrolyl, triazolyl, tetrazolyl, isothiazolyl, quinolyl, isoquinolyl, quinazolinyl, phthalazinyl, indolyl, benzothiazolyl, benzoimidazolyl, benzofuranyl, indazolyl, isoindolyl and benzothienyl.

The benzene-fused heteroaryl groups include, for example, quinolyl, isoquinolyl, quinazolinyl, phthalazinyl, indolyl, benzothiazolyl, benzoimidazolyl, benzofuranyl, indazolyl, isoindolyl and benzothienyl.

The heterocyclic groups include, for example, pyridyl, pyrimidyl, pyridazinyl, pyrazinyl, imidazolyl, isoxazolyl, pyrazolyl, oxazolyl, thiazolyl, furyl, thienyl, pyrrolyl, triazolyl, tetrazolyl, isothiazolyl, quinolyl, isoquinolyl, quinazolinyl, phthalazinyl, indolyl, benzothiazolyl, benzoimidazolyl, benzofuranyl, indazolyl, isoindolyl, benzothienyl, pyrrolidinyl, piperidinyl, piperazinyl, morpholinyl, tetrahydropyranyl and thiazolidinyl.

The halogen atoms include fluorine, chlorine, bromine and iodine.

The alkoxyl groups include, for example, methoxyl, ethoxyl, propoxyl, isopropoxyl, butoxyl, isobutoxyl, secondary butoxyl, tertiary butoxyl, pentyloxyl, isopentyloxyl, neopentyloxyl, tertiary pentyloxyl and hexyloxyl groups.

The substituents of the substituted amino groups include, for example, lower alkyl, $C_{3-7}$ cycloalkyl, aryl, heteroaryl, aralkyl and aryl-lower alkyl groups.

The acyl groups include, for example, acetyl, propionyl, butyryl, isobutyryl, pivaloyl, hexanoyl and cyclohexylcarbonyl groups.

The alkyl groups include, for example, methyl, ethyl, propyl, isopropyl, butyl, isobutyl, secondary butyl, tertiarybutyl, pentyl, isopentyl, neopentyl, tertiary pentyl and hexyl groups.

The cycloalkyl groups include, for example, cyclopropyl, cyclobutyl, cyclopentyl and cyclohexyl groups.

The aralkyl groups include, for example, benzyl and methoxybenzyl groups.

The alkenyl groups include, for example, vinyl, propenyl, butenyl and pentenyl groups.

The alkyl, alkenyl and alkoxyl groups may be either linear or branched chain. When these groups are contained in other groups as a component thereof, the above-described definition is also applied to them.

The compounds of the present invention are usable as therapeutic agents for irritable bowel syndrome of diarrhea type, ulcerative colitis, visceral pain and abdominal pain.

The "irritable bowel syndrome of diarrhea type" herein indicates a disease belonging to "functional gastrointestinal disorders" having chronic or repeated functional gastrointestinal troubles which cannot be explained with reference to the organic or biochemical abnormality. Symptoms of mainly abdominal pain and diarrhea last for a certain period of time [Rome II; the functional gastrointestinal disorders, 2nd Ed., Degnon Associates, McLean (2000), Gastroenterol. Internat., 3, 159-172 (1990)].

The "ulcerative colitis" herein indicates an inflammatory disease of unknown etiology, and patients with this disease have an inflammation in the membrane of the large intestine and an erosion (shallow ulcer and sore) and ulcer on the most inside layer of this membrane. The patients have symptoms of diarrhea, viscous bloody stool, etc. and they repeat the remission and deterioration to elongate the stage [Manual of Gastroenterology, 2nd Ed., Little, Brown and Company 233, 246 (1994)].

The "visceral pain" herein indicates a pain in the internal organs such as the stomach, intestinal tracts and heart as well as the peritonea and pleurae [Textbook of Pain, 4nd Ed., 603-709, CHURCHILL LIVINGSTON, Hartcourt Publishers Limited (1999)].

The "abdominal pain" herein indicates a chronic or acute pain in the abdomen [Textbook of Pain, 4nd Ed., 603-619, CHURCHILL LIVINGSTONE, Hartcourt Publishers Limited (1999)].

The "therapeutic agents for irritable bowel syndrome of diarrhea type" in the present invention are usable not only for the treatment but also for the improvement and prevention of the irritable bowel syndrome of diarrhea type.

The "therapeutic agents for ulcerative colitis" herein are usable not only for the treatment but also for the improvement and prevention of the ulcerative colitis.

The "therapeutic agents for visceral pain" herein are usable not only for the treatment but also for the improvement and prevention of the visceral pain.

The "therapeutic agents for abdominal pain" herein are usable not only for the treatment but also for the improvement and prevention of the abdominal pain.

The compounds of the present invention can be used not only alone but also in combination with other medicines such as cholinergic blocking agents,laxatives, antidiarrheal agents, medicines for intestinal disorders, mucous membrane paralyzing agents, motor function regulators for the digestive tract, autonomic nerve regulators, herb medicines, anxiolytic agents, antidepressants, hypnotics, antipsychotic agents, anti-inflammatory agents, adreno-cortical hormone preparations, immunosuppressive agents, analgesic agents, serotonin antagonists excluding 5-HT7 receptor antagonists, and serotonin agonists.

The 5-HT7 receptor antagonists of the invention of the present application can be synthesized by a method described in, for example, EP 0738513, Japanese Patent Kokai No. Hei 11-189585, WO 97/29097, WO 97/48681, WO 97/49695, WO 98/00400, WO 99/24022, WO 99/31062, WO 99/33804, WO 00/00472, WO 00/56712, WO 00/59909, WO 00/69437, WO 00/73299, WO 01/29029, WO 01/57039, WO 01/85701, WO 02/18367, WO 02/36554, WO 02/36560 or WO 02/62788.

Concretely, the compounds described in the patent specifications of the following numbers can be produced by methods described therein: EP 0738513, Japanese Patent Kokai No. Hei 11-189585, WO 97/29097, WO 97/48681, WO 97/49695, WO 98/00400, WO 99/24022, WO 99/31062, WO 99/33804, WO 00/00472, WO 00/56712, WO 00/59909, WO 00/69437, WO 00/73299, WO 01/29029, WO 01/57039, WO 01/85701, WO 02/18367, WO 02/36554, WO 02/36560 or WO 02/62788.

For example, (R)-2-(2-(4-methylpiperidine-1-yl)-ethyl)-1-(naphthalene-1-sulfonyl)pyrrolidine can be obtained by the following method: (R)-2-pyrrolidinemethanol is reacted with di-t-butyl dicarbonate to obtain t-butyl (R)-2-hydroxymethylpyrrolidine-1-carboxylate. Then this compound is reacted with methanesulfonyl chloride to mesylate the hydroxyl group. The obtained product is reacted with sodium cyanate to obtain t-butyl (R)-2-cyanomethylpyrrolidine-1-carboxylate. The obtained compound is subjected to the reductive amination with 4-methylpiperidine in the presence of hydrogen and platinum oxide. The compound thus obtained is treated with trifluoroacetic acid to obtain (R)-2-(2-(4-methylpiperidin-1-yl)-ethyl)pyrrolidine. This compound is condensed with 1-naphthalenesulfonyl chloride in the presence of diisopropylethylamine to obtain the intended compound.

(R)-3-(2-(2-(4-methylpiperidine-1-yl)-ethyl)pyrrolidine-1-sulfonyl)-phenol and (R)-1-bromo-3-(2-(2-(4-methylpiperidine-1-yl)ethyl)pyrrolidine-1-sulfonyl)benzene can be synthesized by a method described in WO 97/48681 in the same manner as that described above.

The compound thus obtained by the above-described method is isolated and purified in the free form or in the form of a salt thereof. The isolation and purification can be conducted by the extraction, concentration, evaporation, crystallization or silica gel column chromatography as described in WO 97/48681.

The pharmacologically acceptable salt of 5-HT7 receptor antagonist used in the present invention can be any of pharmacologically acceptable salts. For example, when the salt contains a basic group, the salt is that with an inorganic acid such as hydrochloric acid, sulfuric acid or phosphoric acid, an organic acid such as acetic acid, citric acid, benzoic acid, maleic acid, fumaric acid, tartaric acid or succinic acid, or an organic sulfonic acid such as methanesulfonic acid or p-toluenesulfonic acid. When the salt contains an acidic group, the salt is that with ammonium, with an alkali metal such as sodium or potassium, with an alkaline earth metal such as calcium or magnesium, with aluminum, with zinc, with an organic amine such as triethylamine, ethanolamine, morpholine or piperidine, dicyclohexylamine, or with a basic amino acid such as arginine or lysine. For forming the salt, the compound of the present invention is mixed with a required acid or base in a suitable quantitative ratio in a solvent or a dispersant or the salt can be formed by the cation exchange or anion exchange depending on the form of the salt.

The 5-HT7 receptor antagonists used in the present invention include the solvates, such as hydrates and alcohol adducts, of them.

In the compounds of the present invention, those having an asymmetric carbon atom may have an optical isomer, which is included in these compounds.

When the compounds of the present invention have diastereomers, these diastereomers and a mixture of them are also included therein.

When the compounds of the present invention have a tautomeric hydrogen atom, various tautomers are possible. The compounds of the present invention also include those tautomers.

When the compound or the pharmacologically acceptable salt of the present invention is used as a therapeutic agent for patients suffering from the irritable bowel syndrome of diarrhea type, it can be suitably mixed with a pharmaceutically acceptable adjuvants such as an excipient, a carrier or a diluent and the obtained mixture is shaped into tablets, capsules, granules, fine granules, a powder, pills, a syrup, a suspension, an emulsion, an ointment, suppositories or an injection and then orally or parenterally administered to the patients. The carriers and diluents usable herein include, for example, glucose, sucrose, lactose, talc, silica, cellulose, methylcellulose, starch, gelatin, ethylene glycol,

polyethylene glycol, glycerol, ethanol, water, oil and fat.

The route of administration may be either oral or parenteral. The dose, which varies depending on the age, body weight and conditions of the patient and also the administration method, is usually 0.01 to 2,000 mg/day, preferably 0.1 to 500 mg/day for adults in the oral administration and 1 μg to 1,000 mg, preferably 0.01 to 100 mg, for adults in the parenteral administration. When the above-described compounds are used as the therapeutic agents for patients with irritable bowel syndrome of diarrhea type, ulcerative colitis, visceral pain or abdominal pain, they are particularly effectively usable in the oral administration.

Example 1

Effect on 5-hydroxytryptophan (5-HTP)-induced defecation models of mice *in vivo*:

The tests were conducted by using (R)-3-(2-(2-(4-methylpiperidine-1-yl)-ethyl)pyrrolidine-1-sulfonyl)phenol (synthesized according to a method described in WO 97/48681) known to be a selective 5-HT7 receptor antagonist as test compound 1 by a method of G. J. Sanger et al. (British Journal of Pharmacology, 130: 706-712, 2000).

Male SLC:ICR mice (6 weeks) were placed in a series of 5 stainless steel cages for mice. After the acclimation for one hour or longer, 30 mg/kg of test compound 1 was orally administered to them (n=10). 30 minutes after, 10 mg/5mL/kg of 5-HTP (or 5 mL/kg of physiological saline in a group in which 5-HTP was not used) was subcutaneously administered to the mice. The stool excreted by each mouse during 30 minutes after the administration was observed (the results were scored as follows: 0: normal stool or no defecation, 1: diarrhea or soft stool). The inhibitory rate (%) of test compound 1 was calculated while the score obtained by subtracting the score of the 5-HTP-free group from the score of the 5-HTP group was determined to be 100 %.

The control rate of the test compound 1 was 70 %.

It is apparent from the results that the 5-HTP receptor antagonist of the present invention can exhibit an excellent effect as a therapeutic agent for the irritable bowel syndrome of diarrhea type.

Example 2

Effect on dextran sulfate sodium (DSS) mouse models:

The tests were carried out by using the test compound 1, and predonisolone (PDL) and salazosulfapyridine (SASP) as the controls by a method of Arai et al. (Dig Dis Sci., 44, 845, 1999).

Female CBA mice (9-10 weeks) were allowed to drink 5 % DSS (M. W. 5000) as they drinked ad libitum for 12 days to cause ulcerative colitis. The medicine was suspended in 0.5 % tragacanth gum solution, and the oral administration of 5 ml/kg of the obtained suspension was started on the day next to the start of 5 % DSS and continued for 11 days. Then 5 mg/kg of 6 mg/ml Evans Blue solution was given to each mouse by the intravenous injection. 30 minutes after, the intestinal tract was isolated and the length of the tract was measured. The intestinal tract was dried over night. The intestinal tract was kept in formamide at 60°C overnight. After the extraction of Evans Blue, the absorbance was determined, and the infiltrated amount of Evans Blue was calculated according to the following formula:

$$\text{The infiltrated amount of Evans Blue } D = (A*C+B)/E$$

wherein the relationship between the absorbance and the infiltrated amount of Evans Blue was calculated by preparing a working curve (Y=A*X+B) and C represents the absorbance of Evans Blue extracted from the intestinal tract. The infiltrated amount of Evans Blue is used as an index of the protein infiltration and the area of the ulcer in the intestinal tract.

The results are shown in Figs. 1 and 2.

In Figs. 1 and 2, the number in the parentheses represent the number of mice. It is apparent from the results that 5-HT7 receptor antagonist in the present invention is capable of exhibiting an excellent effect as a therapeutic agent for ulcerative colitis.

Example 3

Effect on acetic acid induced writhing models of mice:

The tests were carried out with the above-described test compound 1 by a method of Matsumoto et al. (Eur J Pharmacol., 352, 47, 1998).

The effects of the test compound 1 on male ICR mice (4 weeks old) were examined by the writhing test with acetic acid. 0.9 % acetic acid solution (diluted with physiological saline) was given to each mouse by the intraperitoneal injection. The counting of the number of the writhing in 15 minutes was started 5 minutes after the injection. The test compound was suspended in 0.5 % tragacanth gum solution, and 5 ml/kg of the obtained suspension was orally administered 90 minutes before the administration of acetic acid.
The test results are shown in Fig. 3. In Fig. 3, the numbers in the parentheses represent the numbers of mice. In the statistical assay, one-way layout dispersion analysis was followed by Dunnett multiple comparison (*$p<0.05$, **$p<0.01$ VS. control group).
It is apparent from the results that the 5-HT7 receptor antagonist of the present invention can exhibit an excellent effect of a therapeutic agent for visceral pain and abdominal pain.

## Claims

1. A therapeutic agent for irritable bowel syndrome of diarrhea type, which contains a 5-HT7 receptor antagonist or a pharmaceutically acceptable salt thereof as the active ingredient.

2. The therapeutic agent for irritable bowel syndrome of diarrhea type according to claim 1, wherein the 5-HT7 receptor antagonist is a compound represented by the following general formula (II):

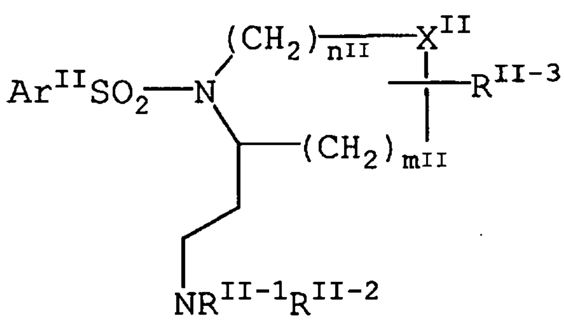

(I I)

wherein:

$Ar^{II}$ represents a substituted or unsubstituted mono- or bicycloaromatic ring or heteroaromatic ring,
$R^{II-1}$ and $R^{II-2}$ independently represent hydrogen, a lower alkyl or an aryl-lower alkyl or, $R^{II-1}$ and $R^{II-2}$ together form a substituted or unsubstituted, 5- to 7-membered heterocyclic ring with the nitrogen atom bonded thereto, which hetero ring may further contain a hetero atom selected from the group consisting of nitrogen, sulfur and oxygen, and the nitrogen atom may be substituted with hydrogen, a lower alkyl or $C_{3-7}$ cycloalkyl or with an aryl, a heteroaryl or an aryl-lower alkyl group,
$R^{II-3}$ represents hydrogen or a lower alkyl,
$X^{II}$ represents oxygen, sulfur or a bond,
$n^{II}$ represents 2 or 3, and
$m^{II}$ represents 1 or 2.

3. The therapeutic agent for irritable bowel syndrome of diarrhea type according to claim 2, wherein the 5-HT7 receptor antagonistis(R)-3-(2-(2-(4-methylpiperidin-1-yl)-ethyl)-pyrrolidine-1-sulfonyl)phenol,(R)-1-bromo-3-(2-(2-(4-methylpiperidine-1-yl)-ethyl)pyrrolidine-1-sulfonyl)-benzene or (R)-2-(2-(4-methylpiperidin-1-yl)-ethyl)-1-(naphthalene-1-sulfonyl)pyrrolidine.

4. A therapeutic agent for ulcerative colitis, which contains a 5-HT7 receptor antagonist or a pharmaceutically acceptable salt thereof as the active ingredient.

**5.** The therapeutic agent for ulcerative colitis according to claim 4, wherein the 5-HT7 receptor antagonist is a compound represented by the general formula (II) in claim 2.

**6.** The therapeutic agent for ulcerative colitis according to claim 5, wherein the 5-HT7 receptor antagonist is (R)-3-(2-(2-(4-methylpiperidin-1-yl)-ethyl)-pyrrolidine-1-sulfonyl)phenol, (R)-1-bromo-3-(2-(2-(4-methylpiperidin-1-yl)-ethyl)pyrrolidine-1-sulfonyl)benzene or (R)-2-(2-(4-methylpiperidin-1-yl)-ethyl)-1-(naphthalene-1-sulfonyl)-pyrrolidine.

**7.** A therapeutic agent for visceral pain or abdominal pain, which contains a 5-HT7 receptor antagonist or a pharmaceutically acceptable salt thereof as the active ingredient.

**8.** The therapeutic agent for visceral pain or abdominal pain according to claim 7, wherein the 5-HT7 receptor antagonist is a compound represented by the general formula (II) in claim 2.

**9.** The therapeutic agent for visceral pain or abdominal pain according to claim 8, wherein the 5-HT7 receptor antagonist is (R)-3-(2-(2-(4-methylpiperidin-1-yl)-ethyl)-pyrrolidine-1-sulfonyl)phenol, (R)-1-bromo-3-(2-(2-(4-methylpiperidin-1-yl)-ethyl)pyrrolidine-1-sulfonyl)benzene or (R)-2-(2-(4-methylpiperidin-1-yl)-ethyl)-1-(naphthalene-1-sulfonyl)-pyrrolidine.

**10.** Use of the 5-HT7 receptor antagonist or the pharmaceutically acceptable salt thereof according to any of claims 1 to 3 for producing the therapeutic agent for irritable bowel syndrome of diarrhea type.

**11.** Use of the 5-HT7 receptor antagonist or the pharmaceutically acceptable salt thereof according to any of claims 4 to 6 for producing the therapeutic agent for ulcerative colitis.

**12.** Use of the 5-HT7 receptor antagonist or the pharmaceutically acceptable salt thereof according to any of claims 7 to 9 for producing the therapeutic agent for visceral pain or abdominal pain.

FIG.1

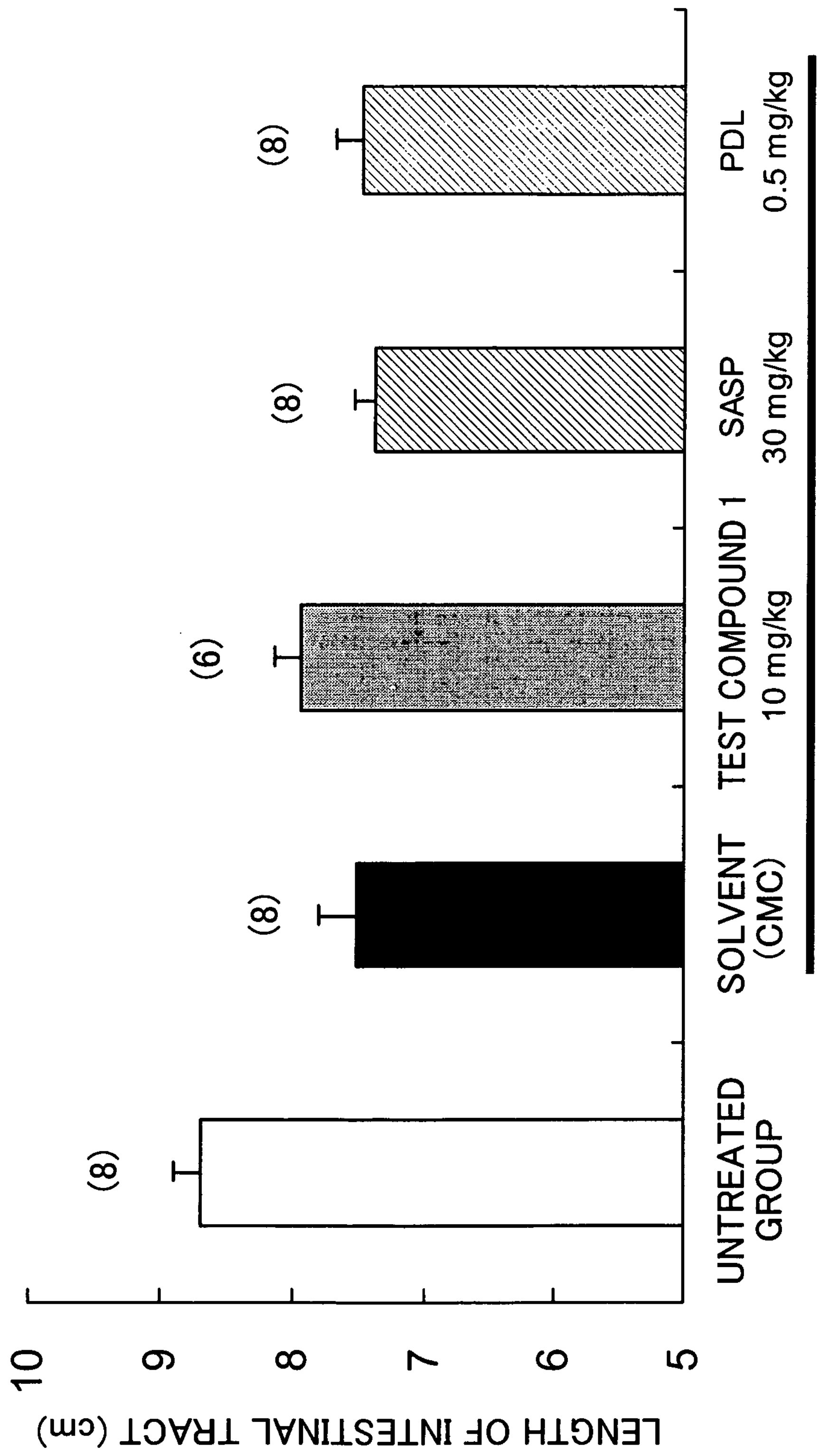
LENGTH OF INTESTINAL TRACT (cm)
10
9
8
7
6
5
(8)
(8)
(6)
(8)
(8)
UNTREATED GROUP
SOLVENT (CMC)
TEST COMPOUND 1 10 mg/kg
SASP 30 mg/kg
PDL 0.5 mg/kg

FIG.2

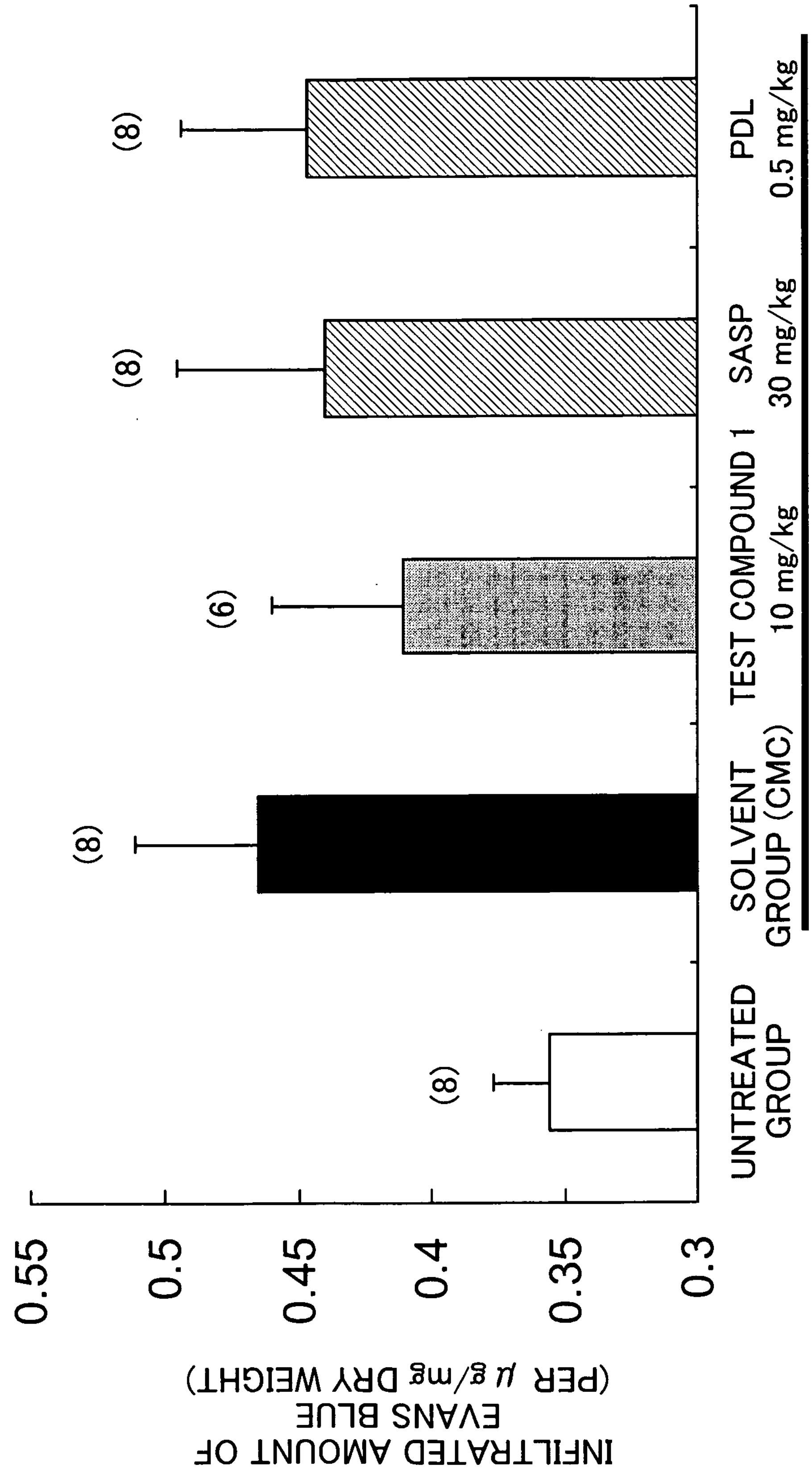
INFILTRATED AMOUNT OF
EVANS BLUE
(PER μg/mg DRY WEIGHT)
0.55
0.5
0.45
0.4
0.35
0.3
(8)
(8)
(6)
(8)
(8)
UNTREATED GROUP
SOLVENT GROUP (CMC)
TEST COMPOUND 1
10 mg/kg
SASP
30 mg/kg
PDL
0.5 mg/kg

FIG.3

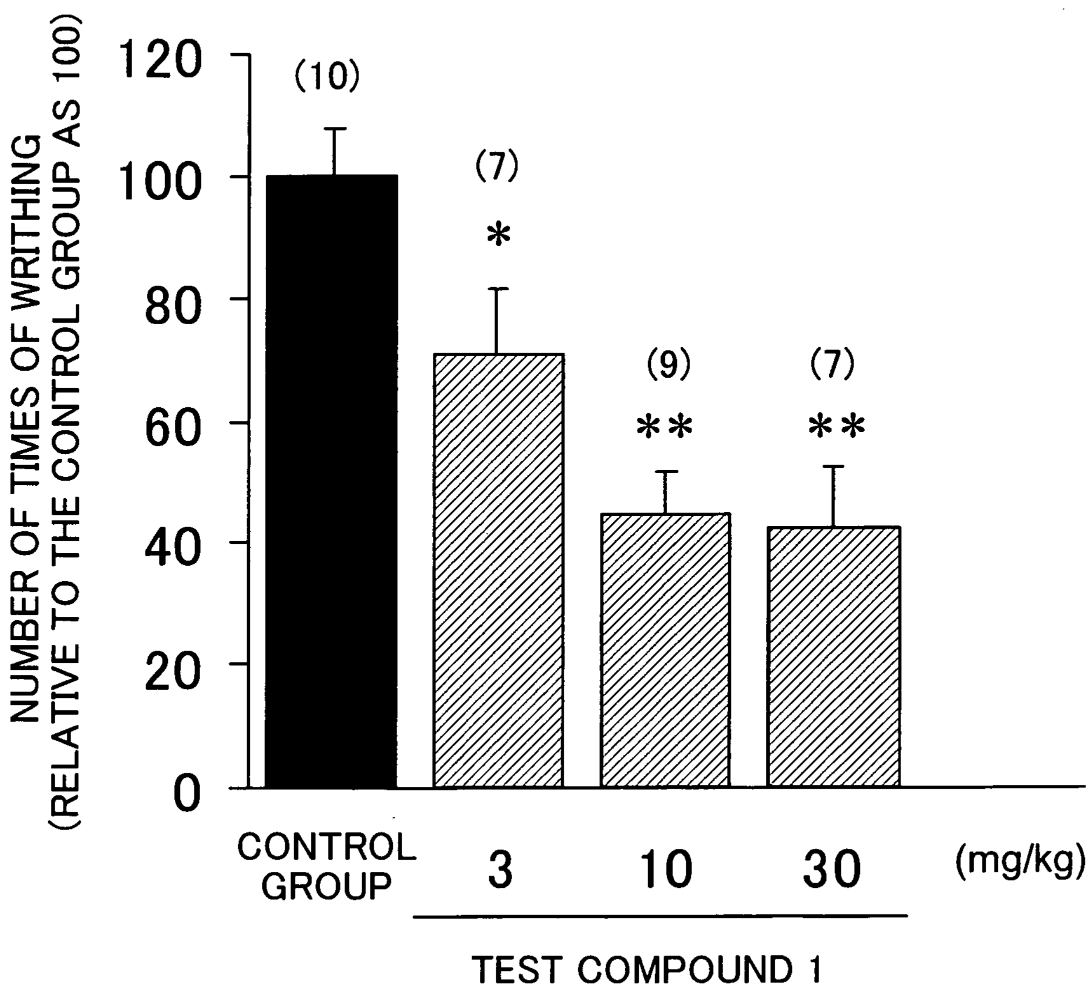
NUMBER OF TIMES OF WRITHING
(RELATIVE TO THE CONTROL GROUP AS 100)
120
100
80
60
40
20
0
(10)
(7)
*
(9)
**
(7)
**
CONTROL GROUP
3
10
30
(mg/kg)
TEST COMPOUND 1

## INTERNATIONAL SEARCH REPORT

International application No.
PCT/JP03/09693

A. CLASSIFICATION OF SUBJECT MATTER
Int.Cl$^7$ A61K45/00, 31/454, A61P1/00, 29/00, 43/00, 1/04

According to International Patent Classification (IPC) or to both national classification and IPC

B. FIELDS SEARCHED
Minimum documentation searched (classification system followed by classification symbols)
Int.Cl$^7$ A61K45/00, 31/454, A61P1/00, 29/00, 43/00, 1/04

Documentation searched other than minimum documentation to the extent that such documents are included in the fields searched

Electronic data base consulted during the international search (name of data base and, where practicable, search terms used)
CAPLUS(STN), REGISTRY(STN), MEDLINE(STN), BIOSIS(STN)

C. DOCUMENTS CONSIDERED TO BE RELEVANT

| Category* | Citation of document, with indication, where appropriate, of the relevant passages | Relevant to claim No. |
|---|---|---|
| A | WO 97/48681 A1 (Smithkline Beecham PLC.), 24 December, 1997 (24.12.97), & JP 12-512645 A & EP 912512 A1 & GB 9612884 A & US 6265408 B1 | 1-12 |
| A | WO 00/73299 A1 (Smithkline Beecham PLC.), 07 December, 2000 (07.12.00), & JP 2003-500488 A & EP 1181287 A1 & US 2003/130275 A1 | 1-12 |
| A | WO 99/24022 A1 (F. Hoffmann-La Roche AG.), 20 May, 1999 (20.05.99), & AU 1558899 A | 1-12 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>04 November, 2003 (04.11.03) | Date of mailing of the international search report<br>18 November, 2003 (18.11.03) |
| Name and mailing address of the ISA/<br>Japanese Patent Office | Authorized officer |
| Facsimile No. | Telephone No. |

Form PCT/ISA/210 (second sheet) (July 1998)

## INTERNATIONAL SEARCH REPORT

International application No.
PCT/JP03/09693

C (Continuation). DOCUMENTS CONSIDERED TO BE RELEVANT

| Category* | Citation of document, with indication, where appropriate, of the relevant passages | Relevant to claim No. |
|---|---|---|
| A | WO 95/09168 A1 (Tokyo Tanabe Co., Ltd.), 06 April, 1995 (06.04.95), & EP 721949 A1 & JP 7-510227 A & US 5677326 A | 1-12 |
| A | EP 623621 A1 (Nisshin Flour Milling Co., Ltd.), 09 November, 1994 (09.11.94), & JP 7-010881 A | 1-12 |
| A | EP 873753 A1 (Pfizer Inc.), 28 October, 1998 (28.10.98), & JP 10-316567 A | 1-12 |
| A | WO 00/31073 A1 (Meiji Seika Kaisha, Ltd.), 02 June, 2000 (02.06.00), & EP 1134220 A1 | 1-12 |
| A | WO 99/17755 A2 (Glaxo Group Ltd.), 15 April, 1999 (15.04.99), & EP 1021174 A1 & US 6284770 B1 & JP 13-518495 A | 1-3,10 |
| A | EP 189002 A2 (Sand AG.), 30 July, 1986 (30.07.86), & JP 61-152628 A | 1-12 |
| A | EP 1031350 A1 (Warner-Lambert Co.), 30 August, 2000 (30.08.00), & WO 00/50027 A1 & JP 2002-537332 A | 7-9,12 |
| A | WO 01/78698 A2 (Warner-Lambert Co.), 25 October, 2001 (25.10.01), & EP 1282421 A2 & JP 2003-530427 A | 7-9,12 |

Form PCT/ISA/210 (continuation of second sheet) (July 1998)

## INTERNATIONAL SEARCH REPORT

International application No.
PCT/JP03/09693

**Box I Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

The inventions according to claims 1 to 3 and 10 relate to a remedy for diarrheal irritable bowel syndrome comprising a 5-HT7 receptor antagonist or its pharmaceutically acceptable salt as the active ingredient. In contrast, the inventions according to claims 4 to 6 and 11 and the inventions according to claims 7 to 9 and 12 relate respectively to a remedy for ulcerative colitis and a remedy for visceral pain or abdominal pain each comprising 5-HT7 receptor antagonist or its pharmaceutically acceptable salt as the active ingredient. However, diarrheal irritable bowel syndrome, ulcerative colitis, visceral pain and abdominal pain are largely different from (continued to extra sheet)

1. ☒ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☒ No protest accompanied the payment of additional search fees.

Form PCT/ISA/210 (continuation of first sheet (1)) (July 1998)

**INTERNATIONAL SEARCH REPORT**

International application No.
PCT/JP03/09693

Continuation of Box No.II of continuation of first sheet(1)

each other in causes and drugs having been employed in practice for treating these diseases. Thus, there is no matter seemingly being a special technical feature in the meaning within the second sentence of PCT Rule 13.2 common to these groups of inventions and there is no technical relevancy in the meaning within PCT Rule 13 among these inventions differing from each other.

Such being the case, there is no technical relationship among these groups of inventions involving one or more of the same or corresponding technical features and thus these groups of inventions are not considered as relating to so linked as to form a single general inventive concept.

<Subject of search>

Claims 1, 4 and 7 relate to remedies for diarrheal irritable bowel syndrome, ulcerative colitis and visceral pain or abdominal pain comprising "a 5-HT7 receptor antagonist or its pharmaceutically acceptable salt" as the active ingredient. Although claims 1, 4 and 7 involve any compounds having the 5-HT7 receptor antagonism, it is recognized that only part of the claimed compounds are supported by the description in the meaning within PCT Article 6 and disclosed therein in the meaning within PCT Article 5.

Although the common technical knowledge at the point of the application is taken into consideration, the scope of the "5-HT7 receptor antagonist or its pharmaceutically acceptable salt" cannot be specified. Thus, claims 1, 4 and 7 do not comply with the requirement of clearness under PCT Article 6 too.

Concerning the relationship among the 5-HT7 receptor antagonist, the remedy for diarrheal irritable bowel syndrome, the remedy for ulcerative colitis and the remedy for visceral pain or abdominal pain, therefore, the search was made on the remedy for diarrheal irritable bowel syndrome, the remedy for ulcerative colitis and the remedy for visceral pain or abdominal pain comprising as the active ingredient the compounds specifically cited in the description and specified in claims 2, 3, 5, 6, 8 and 9. Complete search was made on claims 2, 3, 5, 6, 8 and 9.

Form PCT/ISA/210 (extra sheet) (July 1998)